## Europäisches Patentamt

(19) **European Patent Office**

**Office européen des brevets**

(11) Publication number: **0 083 202 B1**

(12) **EUROPEAN PATENT SPECIFICATION**

(45) Date of publication of patent specification: **17.07.85**

(21) Application number: **82306855.6**

(22) Date of filing: **21.12.82**

(51) Int. Cl.⁴: **C 07 C 7/13, B 01 J 8/42, B 01 D 15/02**

(54) Liquid phase adsorption and separation process.

(30) Priority: **28.12.81 US 335099**

(43) Date of publication of application:
**06.07.83 Bulletin 83/27**

(45) Publication of the grant of the patent:
**17.07.85 Bulletin 85/29**

(84) Designated Contracting States:
**AT BE CH DE FR GB IT LI LU NL SE**

(56) References cited:
**GB-A-2 057 910**
**US-A-4 031 156**
**US-A-4 247 987**

(73) Proprietor: **Exxon Research and Engineering Company**
**P.O.Box 390 180 Park Avenue**
**Florham Park New Jersey 07932 (US)**

(72) Inventor: **Reiter, Thomas Agens**
**2418 Oak Bank**
**Kingwood Texas (US)**
Inventor: **Cheben, Joseph Milan**
**16446 Heatherdale Drive**
**Houston Texas (US)**
Inventor: **Hines, Dwight Dee**
**203 Cedar Lane**
**Seabrook Texas (US)**
Inventor: **Wu, Tronze-I**
**8422 Bunker Bend Drive**
**Humble Texas (US)**
Inventor: **Cahn, Robert Paul**
**799 Ridgewood Road**
**Millburn New Jersey 07041 (US)**
Inventor: **Rosensweig, Ronald Ellis**
**34 Gloucester Road**
**Summit New Jersey 07901 (US)**

(74) Representative: **Northover, Robert Frank et al**
**ESSO Chemical Limited Esso Chemical Research Centre P.O. Box 1**
**Abingdon Oxfordshire, OX13 6BB (GB)**

Courier Press, Leamington Spa, England.

**Description**

The present invention relates to a liquid-phase adsorption process for the separation of hydrocarbons using small adsorbent particles in a magnetically stabilized fluid bed. One of the many possible applications of this invention is in separation of isomers of aromatic hydrocarbons.

Separation of aromatic hydrocarbons from their isomers or from aromatic or non-aromatic hydrocarbon feedstocks is conventionally accomplished through the operation of physical means such as distillation of the desired hydrocarbon from a mixture of hydrocarbons, or by the adsorption of the desired hydrocarbon onto one of the many conventional adsorbent materials well known in the separation art.

As an example of a separation using adsorbent materials, separation of aromatic hydrocarbon isomers by elution chromatography has previously been described in a number of patents, as for example, U.S. Patent Numbers 4,029,717; 4,031,151; 4,031,155 and 4,031,156, all of which are restricted to fixed adsorbent beds or to a plurality of fixed adsorbent beds operated in a cyclic manner so as to simulate a moving bed of adsorbent. Most simply described, for purposes of the present invention, elution chromatography is the separation of desired hydrocarbons from a feedstream containing other hydrocarbons, such as specific aromatics from mixed aromatic or non-aromatic hydrocarbons, based upon the same principles as conventional chromatographic analysis, which is a discontinuous process in which a hydrocarbon feedstock is introduced into a charomatographic column containing a fixed bed of adsorbent materials. After a period of time to allow for maximum adsorption of selected components of the feedstock, a chosen eluent or desorbent, which initially is free of the components found in the feed stream, is then fed continually into the column over a period of time, and causes desorption of the adsorbed components of the feedstream, causing various components to travel through the adsorption bed as individual bands or zones at slightly different velocities. The desorbent used is chosen upon its ability to competitively adsorb with the adsorbed component, that is, its ability to bind to the adsorption sites on the adsorbent and thereby free the desired component. If the adsorption bed is long enough, the zones for each individual component will separate from one another sufficiently to recover the component as a relatively pure extract. In order for this to occur, it is important that the adsorbent and desorbent materials be carefully selected for the intended separation.

Adsorption on a commercial scale with fixed adsorbent beds, however, is limited by particle size of the adsorbent material, commonly a zeolite. When adsorbent particles such as 1,6 mm pellets were used in a fixed bed, poor separation took place because, it is thought, of the high resistance to molecular passage of the long macropore paths inside the large adsorbent particles, resulting in flat chromatographic bands, poor resolution of the individual component peaks, and, therefore, poor recovery and purity of the desired hydrocarbon components. When smaller absorbent particles were employed, as for example particles of a size of 0.42—0.84 mm (through a 20 mesh and on a 40 mesh screen (U.S. Standard)), separations became more distinct and the resultant recoveries and purities improved. Also, the superior separation and recovery resulted in a lower desorbent to feed ratio which made the use of the smaller particles of greater economic benefit. However, the use of still smaller particles, less than 0.42 mm (i.e., through a 40 mesh screen (U.S. Standard)), maintained in the fixed bed mode of adsorption results in large pressure drops across the bed. Although perhaps still a feasible separation process, the resultant flow restriction, because of this large pressure drop, results in a low throughput of feed (low fluid velocity).

Although pressure drops experienced with smaller particles could be reduced by using fluidized beds (which would have minimized the pressure drop experienced with fixed bed technology), such use was precluded because of the loss of staging, i.e., loss of efficiency in separation caused by excessive backmixing and channel formation which is characteristic of such fluidized beds.

With the development of magnetically stabilized fluidized bed technology, as discussed in U.S. Patent 4,115,927 by Rosensweig, the use of fluidized beds which have been magnetically stabilized against excessive backmixing has been found. The Rosensweig patent discloses fluidizing a bed of magnetizable material and subjecting the bed to a uniform magnetic field, with the bed fluidized by a flow of fluid at a velocity between: the minimum fluidization superficial fluid velocity and less than the velocity required to cause time varying fluctuation of pressure difference through the fluidized bed. The resultant magnetically stabilized fluidized bed experiences no gross solids circulation or backmixing and very little or no gas bypassing (bubble formation). While the Rosensweig patent broadly discloses magnetically stabilized fluidized beds for fluids of gas or liquids, its specific teachings are directed to gas phase systems, with the viscosity drag of a liquid phase system and the resultant backmixing being originally thought to be too excessive to provide an economical liquid phase system. Further, with regard to hydrocarbon adsorption separation processes, since the macropore diffusivity of a liquid phase system is much lower (about $10^{-5}$ cm²/sec at room temperature) versus a gas phase system ($10^{-2}$ cm²/sec at room temperature) the column height required to achieve adsorptive separation in a liquid phase magnetically stabilized fluidized bed system was thought to be excessive.

The Coulaloglou et al patent (US Patent 4,247,987) discloses operating a magnetically stabilized fluidized bed by moving the solids in a plug-flow manner countercurrent to the ascending flow of fluidizing medium, with continuous solids addition and removal from the vessel. The bed particles are stabilized against gross solids recirculation and very little or no gas bypassing and the process is disclosed as useful for separation processes. The bed is operated at or near the locus of transition between the bubbling and

2

stabilized region to obtain bed solids of greater fluidity to facilitate movement of solids within the vessels and transfer from vessel to vessel. The separation process uses two vessels or two zones, one each for adsorption and desorption.

By the process of the present invention, there is an improvement in liquid phase hydrocarbon separation from a feedstream of mixed hydrocarbon components. The process involves providing in a column a magnetically stabilized bed of magnetizable adsorbent particles which are fluidized by the flow of liquid through the bed at a flow velocity of from 5 to 3000 mm/minute, applying a magnetic field to the bed to stabilize the orientation of the bed so that staging is preserved and backmixing is suppressed, the field strength being from 50 to 1000 oersted, absorbing at least a portion of the components of the liquid feedstream of hydrocarbons by passing the feedstream through the bed, and desorbing the adsorbed components with a desorbent. Efficiency of separation of the hydrocarbon components is increased by the use of adsorbent particles which are capable of selectively adsorbing components from the feedstream and which pass through a sieve with 0.42 mm openings (40 mesh screen, US Standard), while being able to maintain high liquid velocity (throughput) but without the high pressure drops which hampered previous fixed bed processes. The column allows the feedstream and the desorbent to pass through the bed in the column.

The invention will be described in more detail, though only by way of illustration, with reference to the accompanying drawings, in which:—

Fig. 1 represents an adsorption column used in the process according to the present invention;

Fig. 2 represents the effect of desorbent concentrations upon separation according to the present invention;

Fig. 3 represents the comparison of resolution and slug size for the process according to the present invention, and, that of a conventional fixed bed process;

Fig. 4 represents the effect of bed height and particle size on resolution for the process according to the present invention, and that of a conventional fixed bed process;

Fig. 5 represents the comparison of elution volume and bed height for the process according to the present invention and that of a conventional fixed bed process; and

Fig. 6 represents improved multiple separation with lower elution volume obtained by the process according to the present invention compared to conventional fixed bed technology.

The present invention concerns a liquid phase process for the adsorption separation of a liquid hydrocarbon feedstream, and particularly for the adsorption separation of aromatic hydrocarbons. It may be used to separate aromatic isomers.

Among those hydrocarbons which may be separated by the liquid phase process according to the present invention, for example, are the separation of paraxylene and/or ethylbenzene from a mixed $C_8$ aromatic feedstock; pseudocumene from a feedstock of $C_9$ aromatics; styrene from a mixture of styrene and ethylbenzene; linear paraffins from petroleum naphthas and distillates; linear olefins from a mixture with linear paraffins; and aromatics from a mixture with linear paraffins.

Several resistances to separation are present if one examines the physical process of adsorption. For example, in the separation of paraxylene, the paraxylene to be adsorbed on an adsorbent material must migrate from the bulk fluid to the particle surface across a laminar film by way of external molecular diffusion. Once it is at the surface of the adsorbent particle, the paraxylene enters the particle through the macropores (macropore diffusivity) in the particle. The paraxylene enters crystallites of an adsorbent material, such as zeolite, via micropore diffusion and adsorbs onto the zeolite inner surface. If the adsorbent is present in the form of agglomerates of crystallites then there is an additional diffusion step through agglomerate pores intermediate in size between the macropores and the micropores. While this movement of desired adsorbable molecules is occurring, counter-diffusion of desorbent or prior adsorbed molecules is adding to the mass transfer resistance. Desorption occurs in the opposite direction.

The macropore diffusivity of the adsorbent particles is inversely proportional to the column bed height required to achieve separation. In a liquid phase separation process the macropore diffusivity ($cm^2$/sec) is on the order of $10^{-3}$ times smaller than a gas phase separation process (e.g., separation of xylene isomers at 50°C and at atmospheric pressure) which would indicate that a liquid phase system would require excessive column bed heights. Additionally, the higher fluid viscosity (centipoise) of a liquid phase system versus a vapor phase system (about $10^2$ times greater) would indicate an excessive column bed height would be needed to effect separation. However, by critical control of other parameters, i.e., adsorbent particle size, liquid velocity, and temperature, adsorptive separation in an economical manner with a reasonable bed height for liquid phase fluidized magnetically stabilized beds can be achieved.

The following characteristic mass balance equation of the physical adsorption process (first discussed by M. Kubin, "Deidraq Zur Theorie Der Chromatographic", Coll. Czechzlov. Chem Commun., Vol. 30, pages 104—118, 1965) demonstrates how these resistances affect product peak width and product peak height of the separated component.

$$\frac{\sigma^2}{2\,\mu^2} = \left[\frac{D_L}{V \cdot L}\right] + \left[\frac{E}{1-E}\right]\left[\frac{V}{L}\right] \quad \left[\frac{R_P}{3K_F} + \frac{R_P^2}{15\,D_P\,\theta} + \frac{R_c^2}{15\,D_c K_P}\right]$$

| Measure of Peak Weight, Spreading | | Film Resistances on External Mass Transfer Resistance | Macropore Resistance | Micropore Resistance |

In the equation, the symbols are defined as follows:

$\sigma$=Standard Deviation, 2nd Moment of Peak
$\mu$=Mean, 1st Moment of Peak
$D_L$=Axial Dispersion Coefficient
$E$=Bed Voidage
$V$=Interstitial Velocity
$L$=Bed Height
$R_P$=Radius of Particle
$K_F$=Fluid Phase Mass Transfer Coefficient
$D_P$=Pore Diffusivity
$R_C$=Crystalline Radius
$D_C$=Intracrystalline Diffusivity
$K_P$=Function of Crystalline Equilibrium Constant
$\theta$=Intraparticle Voidage

Film resistance and macropore resistance (macropore diffusivity) have been determined over the years to give the greatest resistances to adsorption. Both of these resistances are reduced substantially according to the present invention by utilizing smaller particles magnetically held in place.

Once the hydrocarbon component of the feedstocks is adsorbed onto the particular adsorbent material, it is removed by the use of a selected desorbent. This desorbent is a material which is capable of displacing the sorbate components of the feedstock material. The desorbent selected may be diluted to obtain the desired strength relative to the hydrocarbon being separated. If the diluent is not adsorbed, then the combined desorbent plus diluent stream is most precisely described as eluent. However, as many nominal diluents may themselves actually serve as extremely weak desorbents, for the purposes of the present invention the combined stream will be referred to as desorbent. For example, the desorbent used in the separation of xylene isomers may be a mixture of toluene and carrier, such as $C_{10}$ to $C_{14}$ linear paraffins; toluene acts by competing with the xylene isomers (or other feed components) for the active sites. Among the suitable desorbents which are useful in the present invention, and particularly useful in the separation of isomeric $C_8$ aromatic hydrocarbons, are toluene, m-diisopropylbenzene, p-diethylbenzene, mixtures of diethylbenzene isomers, o-dichlorobenzene, and the like. This list, of course, is not all encompassing; other desorbents may be selected provided that they are capable of displacing the sorbent components of the feedstock material.

The process of the present invention uses adsorbent particles which are magnetizable and fluidizable. The adsorbent particles may be a composite of an adsorbent sieve, a magnetizable material, that is a material which is magnetic in an externally applied magnetic field or magnetic per se, and, if needed, a binder. The magnetizable material can be a ferromagnetic substance including iron, nickel, cobalt, such as for example, a 50 micron or smaller stainless steel powder.

One example of preparing a composite of the magnetizable material and the adsorbent is described as follows: the magnetizable material such as stainless steel particles and the adsorbent, e.g., the adsorbent sieve, are admixed with a base (matrix or binder) for the adsorbent and a relatively homogeneous gel is formed. The gel may then be dried, calcined and/or sized. Suitable techniques for sizing and shaping the composite adsorbent include extrusion, pilling, beading, spray drying, etc. The magnetizable component may also be composited with the adsorbent by impregnation, cogelling, coprecipitation, etc. The particles may be of any shape, e.g., spherical, irregular shaped or elongated. Although the data presented herein as an example of liquid phase separation is based upon a composite containing 25% by weight of stainless steel, other magnetizable materials in other percentages may obviously be used providing that the adsorbent sieve material is capable of binding to, or being bound by, the magnetizable material, and that the magnetizable material is inert to the components of the separation streams.

The adsorbent materials which can be used in the liquid phase separation of hydrocarbons according to this invention are those well known in the art; and are generally chosen to suit the particular feed to be treated and the component of the feed to be separated. For example, in an embodiment of the present invention, the separation of aromatic hydrocarbons, specifically of paraxylene or paraxylene and ethylbenzene from $C_8$ aromatic isomeric feedstreams, which may comprise principally ethylbenzene, paraxylene, orthoxylene, and metaxylene, separation may be carried out by utilizing particlar natural or synthetic crystalline metal aluminosilicate adsorbent materials whose internal pore surfaces are accessible for selective combination of solid and solute. Examples of traditional crystalline metal aluminosilicates useful for separation of xylene isomers by means of the present process include, without limitation, potassium substituted zeolite X or Y, barium substituted zeolite X or Y, rubidium substituted zeolite X, or barium and potassium or rubidium and potassium substituted zeolites. Other examples of useful crystalline metal aluminosilicate sorbents that may be employed in the present invention can be found as described in U.S. Patent Numbers 3,732,325 and 3,374,974.

The adsorbent material exemplified for the selective adsorption and separation of $C_8$ hydrocarbon isomers in the following example of a liquid phase process according to the present invention is a particulate zeolite, faujasite, preferably potassium-Y faujasite. Potassium-Y-faujasite (K-Y sieve) is manufactured by a relatively simple exchange procedure of washing commercially available sodium-Y-faujasite (NA-Y sieve) having a silica to alumina ratio ($SiO_2/Al_2O_3$) of less than about 5:1, and preferably about 4.0—4.9:1 (higher ratios are normally detrimental to the separation of paraxylene from other $C_8$ isomers) with a solution of potassium chloride. The potassium ions replace the sodium ions and the resulting sodium chloride is removed by washing in distilled water. Potassium-Y-faujasite has been found to be an exceptional adsorbent for separating the xylene isomers; paraxylene being selectively adsorbed in the presence of metaxylene, orthoxylene, and ethylbenzene. The observed order of sorbability for xylene isomers on potassium-Y-faujasite is paraxylene>ethylbenzene>metaxylene>orthoxylene.

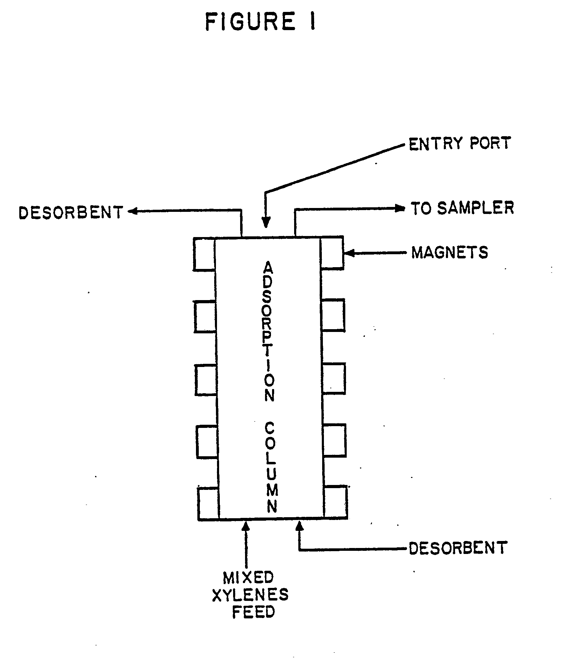

The adsorbent particles are charged into an adsorption column, such as shown in Figure 1, with provisions that will allow the desorbent to travel through the bed. The process configuration of gross solids circulation, if any, and desorbent travel may take various configurations. The process configuration is not critical to the liquid phase process of the present invention, and other configuration may also be used depending upon the optimizing of the specific liquid phase separation to be conducted.

In that instance where the flow of eluent is upwards through the bed, as the flow is increased through the bed, the pressure drop across the bed will rise until a point is reached where the pressure drop across the bed is equal to the weight of the bed per unit area. At this point, the minimum fluidization velocity, enough upward force is present at the bottom of the bed to lift it. As the flow rate is increased, further, the solid composite particles in the bed begin to mix and churn (backmixing) through the bed. The pressure drop across the bed, however, is still approximately equal to the weight of the bed per unit area. The backmixing which occurs in the fluidized bed at this point is not conducive for adsorption and separation of pure components. However, because each adsorbent particle contains a magnetizable material, when the particles are placed within a magnetic field, the particles will assume a stabilized approximate "fixed bed" orientation, free of backmixing in which staging is preserved in a fluidized state. Alternatively, this fixed bed orientation may also be achieved by providing a magnetic field and subsequently fluidizing the particles within the bed, further, if the magnetizable particles are themselves permanently magnetized, a fixed bed orientation may be achieved without application of an external magnetic field.

The application of a magnetic field to the fluidized, expanded or levitated particles containing the magnetizable particles in accordance with the invention is not limited to any specific method of producing the magnetic field. Conventional permanent magnets and/or electromagnets can be employed. The positioning of the magnets will, of course, vary with the solids used, degree of fluidization required and the effects desired. One embodiment of the present invention, for example, a toroidal-shaped electromagnet is employed to surround at least a portion of the fluidized bed; this provides the most uniform magnetic field and consequently the best stability throughout the bed. The electromagnets may be energized by alternating or direct current, with the direct current energized magnetic fields providing lower costs of operation. Such electromagnets when powered by direct current with the use of a rheostat are particularly desirable for applying a magnetic field to the bed particles and to provide an excellent method of stabilizing the fluidization of the bed particles in response to the flow of the fluidizing medium.

The invention is not limited by shape or positioning of the magnet employed to produce the magnetic field. The magnet can be of any size, strength or shape and can be placed above or below the bed to achieve special effects. The magnets employed can be placed within or without the vessel and may even be employed as an integral portion of the vessel structure itself. The process is not limited to any particular vessel material and it can be readily adapted for use in contacting vessels currently employed by industry.

The amount of the magnetic field to be applied to the fluidized adsorbent particles will, of course depend on the desired magnetization for the magnetizable particles and the amount of stabilization desired. Particles having relatively weak magnetic properties, e.g., cobalt, nickel, etc., will require the application of a stronger magnetic field than particulate solids having strong ferromagnetic properties, e.g., iron, to achieve similar stabilization effects. The size and shape of the solids will also obviously have an effect on the strength of the magnetic field to be employed. However, since the strength of the field produced by an electromagnet can be adjusted by adjusting the field strength of the electromagnet, an operator can readily adjust the field strength employed to achieve the desired degree of stabilization for the particular system employed. Generally, the magnetic field strength required to stabilize the bed will be within the range of at least 50 oersted up to 1000 oersted, preferably 200 oersted to 500 oersted.

The magnetically stabilized fluidized bed enables a liquid phase system to use small adsorbent particles, i.e., particles of less than 0.42 mm (which pass through a 40 mesh screen (U.S. Standard)), preferably 0.149 to 0.25 mm (through a 60 mesh screen (U.S. Standard) and on a 100 mesh screen (U.S. Standard)). These small adsorbent particles less than 0.42 mm can be used in a magnetically stabilized fluidized bed while maintaining a substantial fluid velocity through the bed. Since the bed is fluidized, the pressure drop is limited to the weight of the bed per unit area, independent of the liquid throughput (velocity) and particle size (assuming the weight of the bed is constant). Thus, regardless of the particle size and even with smaller particle sizes, a higher liquid velocity rate may be used without suffering the high pressure drops of the previous fixed beds. In addition, the efficiency of the liquid phase separation process is preserved as the magnetically stabilized fluidized bed prevents excessive backmixing and channel formation.

By allowing a liquid phase adsorption and separation process to efficiently and economically employ small adsorbent particles (less than 0.42 mm) increased separation and resolution of separation is achieved, and lower desorbent volume is required versus a fixed bed of larger adsorbent particles. Further since particle size is directly proportional to the column (bed) height needed for separation, the smaller particle size will permit lower column heights to be employed at substantial economic savings.

Additional parameters which are controlled in a liquid phase adsorption and separation process employing magnetically stabilized fluidized beds include: liquid velocity; liquid viscosity and liquid temperature. The liquid velocity through the bed must be sufficient to fluidize the adsorbent particles (minimum fluidization velocity) and generally is within the range of about 5 to 3000 mm/minute, preferably 25 to 500 mm/minute. The viscosity of the liquid is generally within the range of 0.1 to 10 centipoise, preferably 0.1 to 1 centipoise. The temperature of the liquid is controlled to maintain the liquid phase, preferably within the range of ambient temperature to 150°C. Additionally, the pressure of the adsorption column is generally controlled to within the range of 0.97 to 6.9 bar (14 to 100 psia), preferably 0.96 to 2.07 bar (14 to 30 psia), although in a liquid phase system pressure control is not paramount.

Beneficially, the energy consumed in a liquid phase adsorption and separation magnetically stabilized fluidized bed process is surprisingly about 1/3 to 1/2 the amount of energy consumed in a vapor phase adsorption and separation magnetically stabilized fluidized bed process. For example, it is estimated that to recover paraxylene from a mixed feed of xylenes would consume about 4865 BTU/per lb ($11.3.10^6$ J/kg) of paraxylene in a vapor phase process, as compared to 1830 BTU/per lb ($4.3.10^6$ J/kg) of paraxylene in a liquid phase process.

In order to provide a comparison of prior art liquid phase adsorption and separation processes which utilize fixed beds with the liquid phase adsorption and separation process of the present invention, a number of experiments were conducted using the separation of xylene isomers as an example. In each of the following examples demonstrating the advantages of the liquid phase process, according to the present invention, the apparatus used is shown in Figure 1, and the procedure to conduct those series of experiments is described below.

Figure 1 shows an Adsorption Column having a series of magnets located along the outer surface of the column. The bottom of the column is adapted to receive a mixed hydrocarbon feedstream and a desorbent feedstream. The top of the column is adapted to have a sealable entry port in the area generally indicated, for the loading of the magnetizable particles into the column. The top of the column is also adapted to have two outlets, one for excess desorbent while the bed is undergoing fluidization, and another for sample (or product) removal for the determination of operability of the system.

In use, the magnetizable particles were loaded into the adsorption column, (having an internal diameter of 2 inches (5.1 cm) and a length of 9 feet (2.74 m) through the entry port, and the port sealed. As an aid to uniform filling of the column, the column was first filled with desorbent and the particles poured through the entry port into the liquid-filled column. Flow of desorbent was established through the column at a rate of 100 cc/minute. This flow was continued for 60 minutes to completely fluidize the bed. The magnets (in this case the system utilized electromagnets) were then activated for 10 minutes to assure complete stabilization of the bed (for the size of the particles and the flow rate through the column, a magnetic field of 450 oersted was used in these experiments; however, the strength of the field would necessarily be adjusted to the process parameters for different flow rates and particle sizes). In the comparisons which follow, the magnetizable particles for the magnetically stabilized fluidized bed separations had a particle size of 0.149 to 0.25 mm (through a 60 mesh and on a 100 mesh screen (U.S. Standard)) and the particles for the comparison fixed bed separations were larger at 0.42 to 0.84 mm (through 20 mesh on 40 mesh screen (U.S. Standard)). Ambient temperature was used in all experiments.

After stabilization, a 30 cm³ sample of mixed xylenes (40% metaxylene, 20% ethylbenzene, 20% paraxylene, and 20% orthoxylene) was introduced into the bottom of the column where indicated. Samples of the overhead were taken every 2 minutes for two hours, analyzed by gas chromatography, and integrated peak areas were plotted against total elution volume (defined as the amount of desorbent necessary to sweep peaks from the column).

As discussed previously, when adsorbent particles of 0.42 mm (through a 40 mesh screen (U.S. Standard)) are employed in the fixed bed mode of adsorption, large pressure drops occur through the bed. In order to compensate for this increase in pressure drop, additional pumping energy and apparatus is required to push the liquid through the bed. When smaller particles are used according to the process of the present invention, however, the pressure drop is essentially the same, as shown in Table 1, as that in the fixed bed process using larger particles. The superficial velocity of the liquid in the column for the data of Table was 60 mm/minute.

TABLE I

| Mode | Bed height | Particle size (mm) | Pressure drop bar |
|------|-----------|--------------------|--------------------|
| Magnetically Stabilized Fluidized Bed | 10 ft (3.05 m) | 0.149—0.177 | 0.21 |
| Fixed Bed | 10 ft (3.05 m) | 0.42 —0.84 | 0.23 |
| Fixed Bed | 10 ft (3.05 m) | 0.149—0.177 | 2.07 |

Thus, the use of smaller particles in the process of this invention results in no appreciable pressure drop increase when compared to the fixed bed of adsorption processes of the prior art. This finding provides a great economical advantage also, for separations are much more efficient with smaller particle sizes. Pressure drop with particles of a size of 0.177 to 0.42 mm (through 40 mesh and on 80 mesh (U.S. Standard)), for example, at a feedstream rate of 300 mm/minute through the fluidized fixed bed orientation according to the present invention is equivalent to the pressure drop obtained with particles of 0.42 to 0.84 mm (through 20 mesh on 40 mesh (U.S. Standard)) when used in conventional fixed bed adsorption process at a feedstream rate of 90 mm/minute.

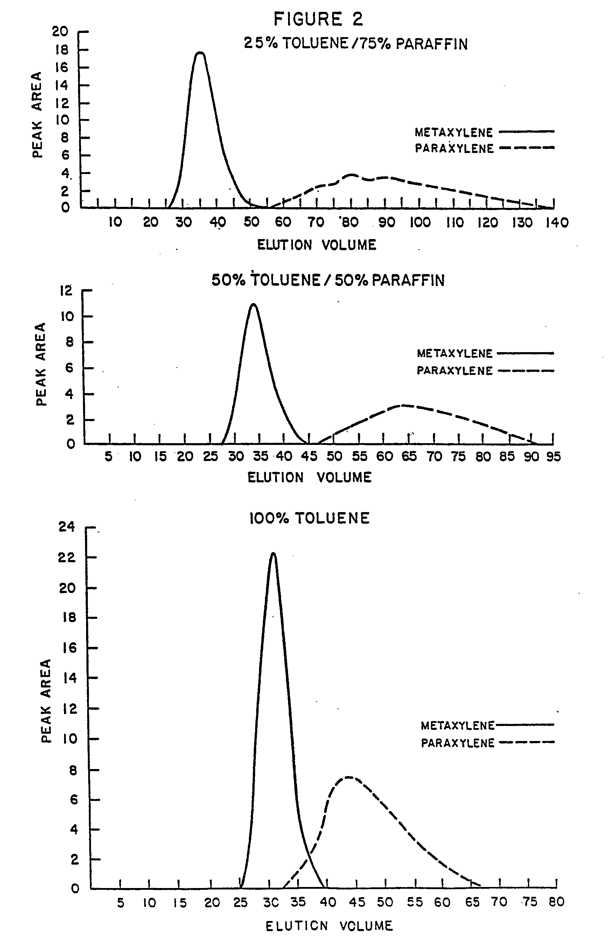

The desorbent selected for the separation of isomeric $C_8$ aromatic hydrocarbons in the following experiments was toluene in a normal linear $C_{10}$—$C_{14}$ paraffin diluent. Separations, according to the present invention, were conducted on a mixed metaxylene-paraxylene feedstream to determine the effect of utilizing various ratios of toluene to diluent at ambient temperature. The results of those tests are graphically shown in Figure 2, wherein the plot on the top of Figure 2 represents the separation of metaxylene from paraxylene using a 25:75 weight percent ratio of toluene to normal linear $C_{10}$—$C_{14}$ paraffins; the middle plot represents the same separation using 50:50 weight percent ratio of toluene to normal linear $C_{10}$—$C_{14}$ paraffins; and the bottom plot represents the same separation usign a desorbent containing only toluene.

The plots of Figure 2 clearly demonstrate the clean separation that occurs around the desorbent concentration of 50:50 weight percent toluene to linear paraffin ratio at ambient temperature. As the toluene concentration decreases from 50 weight percent, the peak widths of metaxylene, and especially paraxylene, increase to a much greater extent than the distance between the peaks increases. This is seen because as the strength of toluene is decreased, it takes a larger volume of desorbent to sweep the peaks out of the column. Thus resolution (and the economic advantage) is decreased. As toluene concentration is increased above 50 weight percent, the distance between the peaks decreases, resulting in less peak separation and lower resolution. This occurs because as the strength of the toluene increases, the peaks are not retained as long by the adsorbent particles and are, therefore, swept out of the column at a faster rate.

Resolution, as used in describing the present invention, is a term of the separation art defined as the desired product recovery at a specific purity level, and is obtained by measuring the distance between two curves at their peaks ($\Delta T$) and between the intersections of each curve at the base line ($W_1$ and $W_2$). Resolution for a two component separation can therefore be shown mathematically as:

$$R = \frac{2 \Delta T}{W_1 + W_2}$$

An R (or resolution) of less than 1.0 indicates a mixing or overlap between the two components (as in Figure 2, bottom curve); an R of 1.0 indicates a complete separation between the two components yielding perfectly symetrical gaussian peaks; and R of more than 1.0 indicates that the products are separated at greater distance along a base line.

Additional experiments were conducted to further quantify the advantages of the present invention over fixed bed.

A series of tests were conducted in order to provide a comparison between the resolution of paraxylene/metaxylene vs. slug size, that is the amount of the mixed component feed introduced into the system, in both the fixed bed and the fluidized magnetically stabilized bed of the present invention. The height of both the fixed and fluidized beds were maintained at 9 feet (2.74 m), and the rate of eluent to the beds was maintained at a flow rate of 103 cc/minute. The magnetizable adsorbent particles used in the fluidized (magnetically stabilized) beds according to the present invention had a particle size of 0.149 to 0.25 mm (through 60 mesh on 100 mesh screen (U.S. Standard)), while the non-ferromagnetic particles in the fixed beds had a particle size of 0.42 to 0.84 mm (through 20 mesh on 40 mesh screen (U.S. Standard)).

The tests confirmed that the results from the adsorption process of the present invention can be interpreted using classical liquid chromatography.

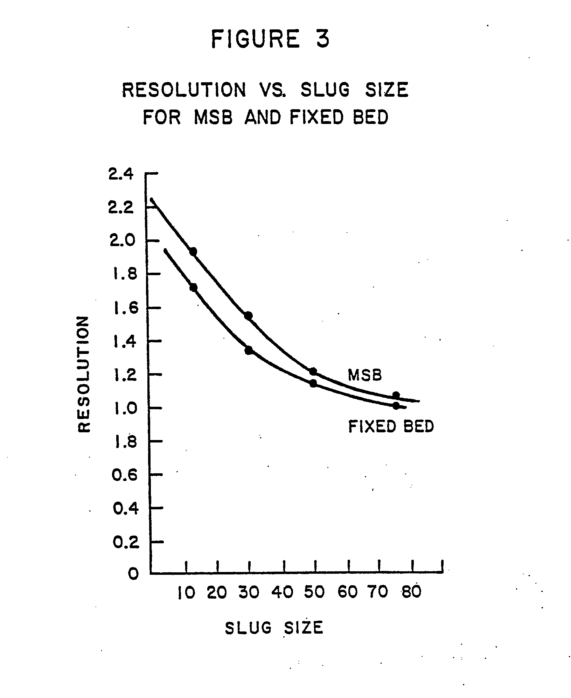

The results of these tests conducted at ambient temperature are shown in Table II, and may be found graphically in Figure 3.

7

## TABLE II

| Slug size cm$^3$ | Resolution in fixed bed | Resolution in fluidized bed |
|---|---|---|
| 15 | 1.70 | 1.92 |
| 30 | 1.32 | 1.58 |
| 50 | 1.15 | 1.21 |
| 75 | 1.02 | 1.05 |

The comparison of the resolution between paraxylene and metaxylene in both the fixed bed and the fluidized bed according to the present invention, clearly shows the improvement at all slug sizes reached by conducting separation in the present process.

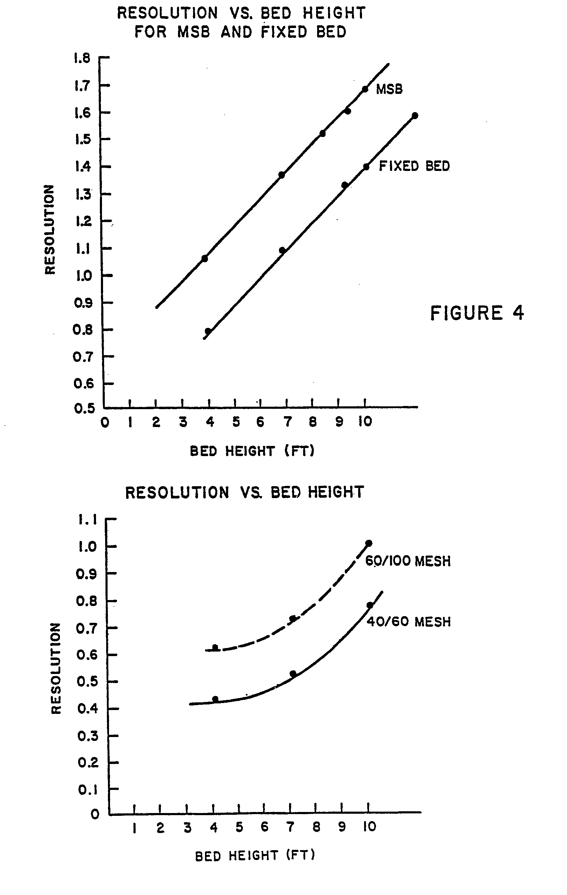

After reviewing the data obtained by comparing the resolution vs. slug size above, a slug size of 30 cc was chosen to compare the effect of bed heights upon the resolution obtained in both fixed and fluidized processes. The results of these tests are shown in Table III, and may be found graphically in Figure 4.

## TABLE III

| Bed height ft (m) | Resolution in fixed bed | Resolution in fluidized bed |
|---|---|---|
| 4 (1.22) | 0.77 | 1.06 |
| 7 (2.13) | 1.09 | 1.37 |
| 8.2 (2.50) | — | 1.48 |
| 9 (2.74) | 1.32 | 1.58 |
| 10 (3.05) | 1.39 | 1.68 |

The comparison of the resolution between paraxylene and metaxylene at ambient temperature in both the fixed bed and the fluidized bed according to the present invention, clearly shows the advantages of the smaller particle size permitted by the fluidized bed of the present invention; at equal bed heights, the resolution obtained in the fluidized bed is much greater than that obtained in the fixed bed.

As discussed previously, when smaller adsorbent particles were employed in the fixed bed adsorption/separation process, separation became more distinct and the resultant recoveries and purities improved. This improvement is also seen in the fluidized process according to the present invention. When resolution is compared with varying bed height for a feed consisting of ethylbenzene and metaxylene in the fluidized bed of the present process; improved resolution is obtained with the use of smaller particles. Data showing such a comparison at ambient temperatures are reported in Table IV, and graphically shown in Figure 4.

## TABLE IV

| Bed height ft (m) | Resolution for 0.25 to 0.42 mm | Resolution for 0.149 to 0.25 mm |
|---|---|---|
| 4 (1.22) | 0.42 | 0.61 |
| 7 (2.13) | 0.51 | 0.71 |
| 10 (3.05) | 0.76 | 0.99 |

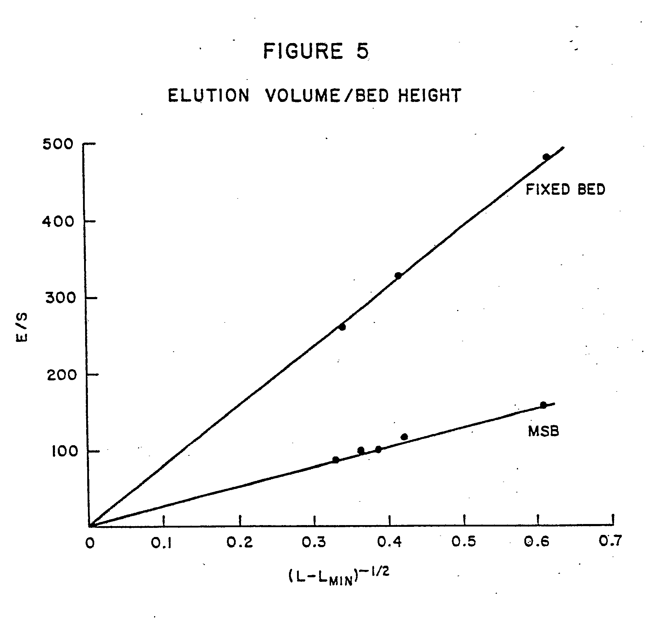

An advantage of the magnetically stabilized fluidized bed process with smaller particles, according to the present invention, over the fixed bed process of adsorption/separation is that the amount of desorbent needed to desorb the adsorbed component is less in the fluidized bed. A comparison between the elution volume/slug size ratio against bed height (L) in both the fixed bed mode and the fluidized bed mode is reported in Table V and graphically in Figure 5 (wherein $L_{min}$ is that minimum bed height required for separation in the fixed bed mode, which has been calculated at 1.3 feet (0.4 m)).

The data presented in Table V based upon a para, meta separation at ambient temperature also follows classical chromatography theory and represents separations at approximately constant resolution.

8

TABLE V

| Bed height (L) ft (m) | $(L-L_{min})^{-1/2}$ | Elution volume/slug size | |
|---|---|---|---|
| | | Fixed bed | Fluidized bed |
| 4 (1.22) | 0.6086 (1.102) | 481 | 170 |
| 7 (2.13) | 0.4189 (0.7588) | 325 | 116 |
| 8.2 (2.50) | 0.3816 (0.6912) | — | 110 |
| 9 (2.74) | 0.3604 (0.6528) | — | 101 |
| 9.7 (2.96) | 0.3457 (0.6262) | — | 88 |
| 10 (3.05) | 0.3390 (0.6140) | 235 | — |

The elution volume/slug size ratio of the fixed bed when compared to that of the fluidized bed (keeping the bed height constant) gives the relative amounts of desorbent needed. For the data contained in Table V, the amount of desorbent needed in the fixed bed mode is calculated at 2.8 to 3.0 times the volume of desorbent needed for the process of the present invention.

The data of Table V and graphically in Figure 5 indicates the relationship between the bed heights for the two processes. If the elution volume/slug size ratio remains constant, in order to achieve equivalent separation the bed height for the fluidized process according to the present invention is 12—14 percent of the bed height required for the fixed bed.

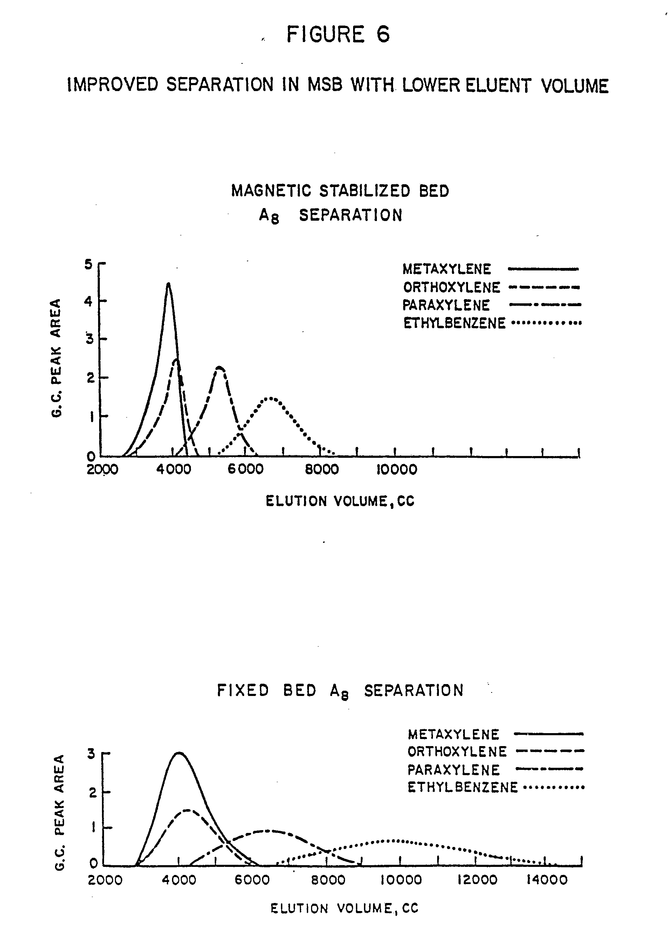

As depicted in Figure 6, the liquid phase adsorption process of the present invention is also capable with smaller adsorbent particles and equal bed heights, of achieving multi-component separations with equivalent or better separation than that obtainable using a fixed bed system. Also, the separations in the liquid phase process of the present invention require less desorbent, that is a lower elution volume, than that required for the fixed bed adsorption process.

Thus, while we have illustrated and described our invention and the manner and process of using it in such full, clear, concise, and exact terms and to enable any person skilled in the art to which it pertains to use the same, one skilled in the art can easily ascertain the essential characteristics of this invention and without departing from the spirit, and scope thereof can make various changes and/or modifications to the invention for adapting it to various usages and conditions. Accordingly, such changes and/or modifications are properly intended to be within the full range of equivalents of the following claims:

## Claims

1. A process for the selective separation of hydrocarbon components from a feedstream containing mixed hydrocarbon components which comprises providing in a column a magnetically stabilized bed of magnetizable adsorbent particles which are fluidized by the flow of liquid through the bed at a flow velocity within the range of 5 to 3000 mm/minute, said adsorbent particles being capable of selectively adsorbing said components from said feedstream and said particles of a size which passes through a sieve with 0.42 mm openings, adsorbing at least a portion of said components from said feedstream by passing said feedstream through said magnetically stabilized fluidized bed, and desorbing said components with a desorbent from said fluidized bed characterized in that:

the process is carried out in the liquid phase;

the column is adapted to allow the feedstream and the desorbent to pass through the bed so that adsorption and desorption are carried out in the column; and

the fluidized bed is magnetically stabilized by applying a magnetic field to said bed at a strength sufficient to maintain said bed in an orientation in which staging is preserved and backmixing is suppressed, said magnetic field strength within the range of from 50 oersted up to 1000 oersted.

2. The process according to Claim 1 wherein the magnetizable adsorbent particles are a composite comprising a magnetizable material and a crystalline metal aluminosilicate.

3. The process according to Claim 2 wherein the magnetizable material is a stainless steel powder.

4. The process according to Claim 2 wherein the crystalline metal aluminosilicate is potassium substituted zeolite X, potassium substituted zeolite Y, barium substituted zeolite X, barium substituted zeolite Y, rubidium substituted zeolite X, barium and potassium substituted zeolites, or potassium and rubidium substituted zeolites.

5. The process according to Claim 4 wherein the crystalline metal aluminosilicate is potassium-Y faujasite.

6. The process of Claims 1—5 wherein the hydrocarbon components contain aromatic hydrocarbon components.

**0 083 202**

7. The process according to Claim 6 wherein said aromatic hydrocarbon components are selected from the group consisting of xylenes and ethylbenzene.

8. The process according to Claim 1, wherein the desorbent is a compound selected from the group consisting of toluene, m-diisopropylbenzene, paradiethylbenzene, mixtures of diethylbenzene isomers, ortho-dichlorobenzene, mixtures of the same, or mixtures with a normal linear paraffin.

9. The process of Claim 2 wherein the adsorbent particles are a size through a sieve with 0.25 mm openings and on a sieve with 0.149 mm openings.

10. The process of Claim 1 wherein the magnetic field strength applied to the bed is within the range of 200 oersted to 500 oersted.

**Patentansprüche**

1. Verfahren zur selektiven Trennung von Kohlenwasserstoffbestandteilen aus einem Beschickungsstrom, der gemischte Kohlenwasserstoffbestandteile enthält, durch Bereitstellung einer magnetisch stabilisierten Schicht aus magnetisierbaren Adsorbensteilchen, welche durch die Strömung von Flüssigkeit durch die Schicht mit einer Strömungsgeschwindigkeit im Bereich von 5 bis 3 000 mm/Min fluidisiert werden, in einer Säule, wobei diese Adsorbensteilchen fähig sind, diese Bestandteile aus diesem Beschickungsstrom selektiv zu adsorbieren und diese Teilchen eine Größe aufweisen, die durch ein Sieb mit Öffnungen von 0,42 mm hindurchgeht, Adsorption mindestens eines Teils dieser Bestandteile aus diesem Beschickungsstrom durch Hindurchführen dieses Beschickungsstromes durch dieses magnetisch stabilisierte Fließbett, und Desorption dieser Bestandteile mit einem Desorbens aus diesem Fließbett, dadurch gekennzeichnet, daß:

das Verfahren in der flüssigen Phase durchgeführt wird;

die Säule angepaßt ist, um dem Beschickungsstrom und dem Desorbens zu ermöglichen, die Schicht derart zu durchlaufen, daß eine Adsorption und Desorption in der Säule durchgeführt werden; und

das Fließbett magnetisch durch Anlegen eines magnetischen Feldes an diese Schicht bei einer ausreichenden Stärke stabilisiert wird, daß diese Schicht in einer Orientierung aufrechterhalten wird, in welcher das Gerüst erhalten bleibt und eine Rückmischung unterdrückt wird, wobei die magnetische Feldstärke in dem Bereich von 50 Oersted bis zu 1 000 Oersted liegt.

2. Verfahren gemäß Anspruch 1, worin die magnetisierbaren Adsorbensteilchen eine Zusammensetzung sind, welche ein magnetisierbares Material und ein kristallines Metallaluminosilikat umfaßt.

3. Verfahren gemäß Anspruch 2, worin das magnetisierbare Material ein Pulver aus rostfreiem Stahl ist.

4. Verfahren gemäß Anspruch 2, worin das kristalline Metallaluminosilikat durch Kalium substituierter Zeolith X, durch Kalium substituierter Zeolith Y, durch Barium substituierter Zeolith X, durch Barium substituierter Zeolith Y, durch Rubidium substituierter Zeolith X, durch Barium und Kalium substituierte Zeolithe oder durch Kalium und Rubidium substituierte Zeolithe sind.

5. Verfahren gemäß Anspruch 4, worin das kristalline Metallalumosilikat Kalium-Y-Faujasit ist.

6. Verfahren nach den Ansprüchen 1 bis 5, worin die Kohlenwasserstoffbestandteile aromatische Kohlenwasserstoffbestandteile enthalten.

7. Verfahren gemäß Anspruch 6, worin diese aromatischen Kohlenwasserstoffbestandteile ausgewählt sind aus der Gruppe bestehend aus Xylolen und Ethylbenzol.

8. Verfahren gemäß Anspruch 1, worin das Desorbens eine Verbindung ausgewählt aus der Gruppe bestehend aus Toluol, m-Diisopropylbenzol, Paradiethylbenzol, Gemischen aus Diethylbenzol isomeren, Orthodichlorbenzol, Gemischen derselben oder Gemischen mit einem normalen linearen Paraffin ist.

9. Verfahren nach Anspruch 2, worin die Adsorbensteilchen von einer Größe durch ein Sieb mit Öffnungen von 0,25 mm und auf einem Sieb mit Öffnungen von 0,149 mm sind.

10. Verfahren nach Anspruch 1, worin die an das Bett angelegte magnetische Feldstärke im Bereich von 200 Oersted bis 500 Oersted liegt.

**Revendications**

1. Procédé de séparation sélective de composants hydrocarbonés d'une charge contenant un mélange d'hydrocarbures, qui consiste à établir dans une colonne un lit, stabilisé par des moyens magnétiques, de particules de matière adsorbante aimantable qui sont fluidisées par l'écoulement du liquide à travers le lit à une vitesse d'écoulement comprise dans l'intervalle de 5 à 3000 mm/min, lesdites particules de matière adsorbante étant capables d'adsorber sélectivement lesdits composants de ladite charge et ces particules ayant un diamètre qui leur fait traverser un tamis de 0,42 mm d'ouverture de maille, à adsorber au moins une portion desdits composants de ladite charge en faisant passer cette dernière à travers le lit fluidisé stabilisé par des moyens magnétiques et à désorber lesdits composants avec une matière désorbante dudit lit fluidisé, caractérisé en ce que:

le procédé est mis en oeuvre en phase liquide;

la colonne est conçue pour permettre le passage de la charge et de la matière désorbante à travers le lit de manière qu'une adsorption et une désorption soient effectuées dans la colonne; et

le lit fluidisé est stabilisé par un champ magnétique qui lui est appliqué avec une intensité suffisante

10

pour maintenir le lit dans une orientation dans laquelle l'échelonnement est préservé et le mélange en retour est supprimé, ladite intensité de champ magnétique étant comprise dans un intervalle allant de 50 à 1000 oersteds.

2. Procédé suivant la revendication 1, dans lequel les particules de matière adsorbante aimantable consistent en un composite comprenant une matière aimantable et un aluminosilicate métallique cristallin.

3. Procédé suivant la revendication 2, dans lequel la matière aimantable est de l'acier inoxydable en poudre.

4. Procédé suivant la revendication 2, dans lequel l'aluminosilicate métallique cristallin est une zéolite X substituée avec du potassium, une zéolite Y substituée avec du potassium, une zéolite X substituée avec de baryum, une zéolite Y substituée avec du baryum, une zéolite X substituée avec du rubidium, des zéolites substituées avec du baryum et du potassium ou des zéolites substituées avec du potassium et du rubidium.

5. Procédé suivant la revendication 4, dans lequel l'aluminosilicate métallique cristallin est la faujasite Y substituée avec du potassium.

6. Procédé suivant les revendications 1 à 5, dans lequel les composants hydrocarbonés contiennent des composants hydrocarbonés aromatiques.

7. Procédé suivant la revendication 6, dans lequel lesdits composants hydrocarbonés aromatiques sont choisis dans le groupe comprenant des xylènes et l'éthylebenzène.

8. Procédé suivant la revendication 1, dans lequel la matière désorbante est un composé choisi dans le groupe comprenant le toluène, le m-diisopropylbenzène le paradiéthylbenzène, des mélanges d'isomères du diéthylbenzène, l'orthodichlorobenzène, des mélanges du même, ou des mélanges avec une paraffine linéaire normale.

9. Procédé suivant la revendication 2, dans lequel les particules de matière adsorbante ont un diamètre tel qu'elles traversent un tamis de 0,25 mm d'ouverture de maille et sont retenues sur un tamis de 0,149 mm d'ouverture de maille.

10. Procédé suivant la revendication 1, dans lequel l'intensité du champ magnétique appliqué au lit se situe dans l'intervalle de 200 à 500 oersteds.

# FIGURE 1

FIGURE 2

0 083 202

2

# FIGURE 3

## RESOLUTION VS. SLUG SIZE
## FOR MSB AND FIXED BED

RESOLUTION VS. BED HEIGHT
FOR MSB AND FIXED BED

FIGURE 4

RESOLUTION VS. BED HEIGHT

# FIGURE 5

## ELUTION VOLUME/BED HEIGHT

X-axis: $(L-L_{MIN})^{-1/2}$

Y-axis: $E/S$

Curves labeled: FIXED BED, MSB

# FIGURE 6

## IMPROVED SEPARATION IN MSB WITH LOWER ELUENT VOLUME

MAGNETIC STABILIZED BED
$A_8$ SEPARATION

MAGNETIC STABILIZED BED $A_8$ SEPARATION

METAXYLENE ——————
ORTHOXYLENE ——————
PARAXYLENE ——·——·——
ETHYLBENZENE ·············

FIXED BED $A_8$ SEPARATION

METAXYLENE ——————
ORTHOXYLENE ——————
PARAXYLENE ——·——·——
ETHYLBENZENE ·············

6